# EUROPEAN PATENT SPECIFICATION

(11) **EP 1 651 181 B1**
(45) Date of publication and mention of the grant of the patent: **27.10.2010**
(21) Application number: 04730542.0
(22) Date of filing: 30.04.2004
(51) Int. Cl.: A61K 8/90, A61K 8/41, A61Q 5/10

(54) **DYEING COMPOSITION COMPRISING AT LEAST ONE DYE PRECURSOR AND ONE AMPHIPHILIC BLOCK COPOLYMER**
FÄRBEMITTEL ENTHALTEND WENIGSTENS EIN FARBSTOFFVORLÄUFERMATERIAL UND EIN AMPHIPHILES BLOCKCOPOLYMER
COMPOSITION DE TEINTURE CONTENANT AU MOINS UN PRECURSEUR DE COLORANT ET UN COPOLYMERE AMPHIPHILE SEQUENCE

(30) Priority: 23.05.2003 FR 0306248; 24.10.2003 US 513594 P
(43) Date of publication of application: 03.05.2006
(73) Proprietor: L'Oréal, 75008 Paris (FR)
(72) Inventor: DUBIEF, Claude, F-78150 Le Chesnay (FR); GUERIN, Frédéric, F-75009 Paris (FR)
(74) Representative: Fevrier, Murielle Françoise E.
(86) International application number: PCT/EP2004/006068
(87) International publication number: WO 2004/103327

(56) References cited:
- FR-A1- 2 805 740
- US-A- 4 183 366
- US-A- 5 690 921
- PATENT ABSTRACTS OF JAPAN vol. 2003, no. 08, 6 August 2003 (2003-08-06) & JP 2003 095898 A (HOYU CO LTD), 3 April 2003 (2003-04-03)
- PATENT ABSTRACTS OF JAPAN vol. 1997, no. 08, 29 August 1997 (1997-08-29) & JP 09 095428 A (NIPPON UNICAR CO LTD), 8 April 1997 (1997-04-08)
- PATENT ABSTRACTS OF JAPAN vol. 1999, no. 11, 30 September 1999 (1999-09-30) & JP 11 171741 A (MANDOM CORP;WAKO PURE CHEM IND LTD), 29 June 1999 (1999-06-29)

## Description

A subject-matter of the invention is a dyeing composition comprising at least one dye precursor and one amphiphilic block copolymer.
Another subject-matter of the invention is the use of this composition for the dyeing of keratinous fibres and the dyeing process employing this composition.

It is known to dye keratinous fibres and in particular human hair with dyeing compositions comprising oxidation dye precursors, generally known as oxidation bases, such as ortho- or para-phenylenediamines, ortho- or para-aminophenols and heterocyclic compounds. These oxidation bases are colourless or weakly coloured compounds which, in combination with oxidizing substances, can give rise by an oxidative coupling process to coloured compounds.

It is also known that the hues obtained with these oxidation bases can be varied by combining them with couplers or colouring modifiers, the latter being chosen in particular from aromatic meta-diamines, meta-aminophenols, meta-diphenols and certain heterocyclic compounds, such as indole compounds.

The variety of the molecules employed as oxidation bases and couplers makes it possible to obtain a rich palette of colours.

The "permanent" colouring obtained by virtue of these oxidation dyes has, however, to satisfy a certain number of requirements. Thus, it must be without disadvantage toxicologically, it must make it possible to obtain hues with the desired intensity and it must behave well in the face of external agents, such as light, bad weather, washing, permanent waving, perspiration and rubbing.

The dyes must also make it possible to cover white hair and, finally, be as unselective as possible, that is to say make it possible to obtain the least possible differences in colouring along the same keratinous fibre, which is generally sensitized (i.e. damaged) to a varying degree between its tip and its root.

The various dyes or dye precursors are generally present in a dyeing medium which conventionally comprises water and optionally a solvent which makes possible better dissolution of the dyes or dye precursors. The medium also conventionally comprises agents, such as anionic, cationic or nonionic, amphoteric or zwitterionic surface-active agents or their mixtures, anionic, cationic, nonionic, amphoteric or zwitterionic polymers or their blends, inorganic or organic thickening agents and in particular anionic, cationic, nonionic and amphoteric associative polymer thickeners. These various constituents are added to the dyeing medium for various reasons, for example to thicken the dyeing medium, to protect, repair or condition the hair before, during or after the colouring, and the like.

The aim of the present invention is to provide novel compositions for dyeing the hair which make it possible to improve the intensity of the colour while retaining a soft feel and ready disentangling after colouring.

This aim is achieved with the present invention, a subject-matter of which is a dyeing composition comprising, in an appropriate medium,
- at least one dye precursor, and
- at least one water-soluble or water-dispersible amphiphilic linear diblock copolymer comprising at least one hydrophilic block and least hydrophobic block according to claim 1 , with the exception of block copolymers of ethylene oxide and of propylene oxide, block copolymers comprising urethane units and block copolymers comprising siloxane units.

Another subject-matter of the invention is the use of the composition of the present invention for dyeing keratinous fibres, in particular human keratinous fibres, such as the hair.

Another subject-matter of the invention is a dyeing process employing this composition.

The composition of the present invention makes it possible in particular to obtain an intense colouring of keratinous fibres while retaining a soft feel and ready disentangling subsequent to the colouring.

The block copolymers which can be used for the present invention are linear diblock copolymers.

The term "water-soluble or water-dispersible block copolymer" is understood to mean, within the meaning of the present invention, a copolymer which, at the concentration of 0.1% of active material in water at 25°C, results, spontaneously or after neutralization with an acid or a base, in a macroscopically homogeneous, transparent or translucent, solution or suspension, that is to say a solution or suspension having a value for the transmission at a wavelength of 500 nm through a sample with a thickness of 1 cm of at least 70%, preferably of approximately 80%.

The term "hydrophobic block" is understood to mean, according to the present invention, a block comprising at least 75 mol% of water-insoluble monomers. In other words, the block can comprise at most 25 mol% of one or more water-soluble monomers according to the definition given above. This proportion is preferably at most equal to 10 mol%. According to a particularly preferred embodiment, this proportion is less than or equal to 5 mol%.

The term "hydrophilic block" is understood to mean a block comprising at least 75 mol% of water-soluble monomers according to the definition given above with reference to the copolymer. In other words, the hydrophilic block can comprise at most 25 mol% of one or more water-insoluble monomers according to the definition given above. This proportion is preferably at most equal to 10 mol%. According to a particularly preferred embodiment, this proportion is less than or equal to 5 mol%.

According to a particularly preferred embodiment, the hydrophilic block or blocks represent at least 60% by weight of the block copolymer.

The water-soluble monomers forming the hydrophilic block of the block copolymers used in the present invention can be chosen from anionic water-soluble monomers, chosen from carboxylic acids comprising ethylenic unsaturation, such as acrylic acid, methacrylic acid, itaconic acid, fumaric acid, crotonic acid and maleic acid anhydride and cationic water-soluble monomers, from N-methyl-4-vinyl--pyridinium.

The water-insoluble monomers forming the hydrophobic block of the block copolymers are chosen from vinylaromatic monomers, such as styrene and its alkylated derivatives, for example 4-butylstyrene, α-methylstyrene and vinyltoluene.

The block copolymers used in the present invention also, of course, encompass those in which the hydrophilic block and the hydrophobic block are composed exclusively of water-soluble monomers and water-insoluble monomers respectively. These blocks can be homopolymer blocks or copolymer blocks including two or more than two different monomers of the same type.

The number-average molecular mass of each block, whether hydrophobic or hydrophilic, copolymer or homopolymer, is preferably between 500 and 100 000, in particular between 500 and 50 000, with a polydispersity index (M_{w}/Mₙ) of between 1.01 and 3.0, preferably between 1.1 and 2.5.

The ratio by weights of the hydrophobic blocks and the hydrophilic blocks of the block copolymer is preferably between 1/20 and 20/1 and in particular between 1/10 and 10/1.

The dyeing compositions of the present invention preferably comprise the diblock copolymers in the dissolved or finely dispersed state in the dyeing medium.

The block polymers of the invention can be prepared by the synthetic processes conventionally used to produce block polymers. Mention may be made, for example, of anionic or cationic polymerizations and controlled radical polymerization (see *"*New Method of Polymer Synthesis", Blackie Academic & Professional, London, 1995, volume 2, page 1. or Trends Polym. Sci., 4, page 183 (1996) by C. J. Hawker), which controlled radical polymerization can be carried out according to different processes, such as, for example, atom transfer radical polymerization (ATRP) (see JACS, 117, page 5614 (1995), by Matyjasezwski *et al*.), or the method of radicals, such as nitroxides (Georges et al., Macromolecules, 1993, 26, 2987).

Use may also be made of these processes to produce just one of the two types of blocks of the polymer of the invention, the other block being introduced into the final polymer via the initiator used or else by coupling reaction between the hydrophilic and hydrophobic blocks.

The amount of amphiphilic block copolymers in the dyeing compositions of the present invention depends on a large number of parameters, among which may be mentioned the molecular mass of the copolymers, the number and size of the hydrophilic and hydrophobic blocks, the amount of polymers which are beneficial for the hair, and the viscosity of the composition which it is desired to obtain.

Generally, the block polymer or polymers are present in the composition of the present invention in an amount of between 0.01 and 20% by weight of active material, preferably between 0.1 and 5% by weight of active material, with respect to the weight of the dyeing composition.

The dye precursors of use in the present invention can be oxidation bases or couplers conventionally used in oxidation dyeing. According to a specific embodiment, the composition of the present invention comprises at least one oxidation base and at least one coupler.

By way of example, the oxidation bases can be chosen from para-phenylenediamines, bisphenylalkylenediamines, para-aminophenols, bis(para-aminophenol)s, ortho-aminophenols, heterocyclic bases and their addition salts.

Mention may be made, among para-phenylenediamines, by way of example, of para-phenylenediamine, para-toluenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-(-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl, β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)-para-phenylenediamine, 4-aminophenylpyrrolidine, 2-thienyl-para-phenylenediamine, 2-(β-hydroxyethylamino)-5-aminotoluene, 3-hydroxy-1-(4'-aminophenyl)pyrrolidine, and their addition salts with an acid.

Among the para-phenylenediamines mentioned above, para-phenylenediamine, para-toluenediamine, 2-isopropyl-para-phenylenediamine, 2-(β-hydroxyethyl)-para-phenylenediamine, 2-(β-hydroxyethyloxy)-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine, 2-(β-acetylaminoethyloxy)-para-phenylenediamine, and their addition salts with an acid, are particularly preferred.

Mention may be made, among bisphenylalkylenediamines, by way of example, of N,N'-bis(β-hydroxyethyl)-N,N'-bis(4'-aminophenyl)-1,3-diaminopropanol, N,N'-bis(β-hydroxyethyl)-N,N'-bis(4'-aminophenyl)-ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane, and their addition salts with an acid.

Mention may be made, among para-aminophenols, by way of example, of para-aminophenol, 4-amino-3-methylphenol, 4-amino-3-fluorophenol, 4-amino-3-(hydroxymethyl)phenol, 4-amino-2-methylphenol, 4-amino-2-(hydroxymethyl)phenol, 4-amino-2-(methoxymethyl)-phenol, 4-amino-(2-aminomethyl)phenol, 4-amino-2-[(β-hydroxyethyl)aminomethyl]phenol, 4-amino-2-fluorophenol, 4-amino-2-chlorophenol, 4-amino-2,6-dichlorophenol, 4-amino-6-[(5'-amino-2'-hydroxy-3'-methylphenyl)methyl]-2-methylphenol and bis(5-amino-2-hydroxyphenyl)methane, and their addition salts with an acid.

Mention may be made, among ortho-aminophenols, by way of example, of 2-aminophenol, 2-amino-5-methylphenol, 2-amino-6-methylphenol, 5-acetamido-2-aminophenol, and their addition salts with an acid.

Mention may be made, among heterocyclic bases, by way of example, of pyridine derivatives, pyrimidine derivatives and pyrazole derivatives.

Mention may be made, among pyridine derivatives, of the compounds disclosed, for example, in Patents GB 1 026 978 and GB 1 153 196, such as 2,5-diaminopyridine, 2-(4-methoxyphenyl)amino-3-aminopyridine, 2,3-diamino-6-methoxypyridine, 2-(β-methoxyethyl)amino-3-amino-6-methoxypyridine, 3,4-diaminopyridine, and their addition salts with an acid.

Other pyridine oxidation bases of use in the present invention are the oxidation bases 3-aminopyrazolo[1,5-a]pyridines or their addition salts disclosed, for example, in Patent Application FR 2 801 308. Mention may be made, by way of example, of pyrazolo[1,5-a]pyridin-3-ylamine; 2-(acetylamino)pyrazolo[1,5-a]pyridin-3-ylamine; 2-(morpholin-4-yl)pyrazolo[1,5-a]pyridin-3-ylamine; 3-aminopyrazolo[1,5-a]pyridine-2-carboxylic acid; 2-methoxypyrazolo[1,5-a]pyridin-3-ylamine; (3-aminopyrazolo[1,5-a]pyridin-7-yl)methanol; 2-(3-aminopyrazolo[1,5-a]pyridin-5-yl)ethanol; 2-(3-aminopyrazolo[1,5-a]pyridin-7-yl)ethanol; (3-aminopyrazolo[1,5-a]pyridin-2-yl)methanol; 3,6-diaminopyrazolo[1,5-a]pyridine; 3,4-diaminopyrazolo[1,5-a]pyridine; pyrazolo[1,5-a]-pyridine-3,7-diamine; 7-(morpholin-4-yl)-pyrazolo[1,5-a]pyridin-3-ylamine; pyrazolo[1,5-a]-pyridine-3,5-diamine; 5-(morpholin-4-yl)-pyrazolo[1,5-a]pyridin-3-ylamine; 2-[(3-aminopyrazolo[1,5-a]pyridin-5-yl)(2-hydroxyethyl)amino]ethanol; 2-[(3-aminopyrazolo[1,5-a]-pyridin-7-yl)(2-hydroxyethyl)amino]ethanol; 3-aminopyrazolo[1,5-a]pyridin-5-ol; 3-aminopyrazolo[1,5-a]pyridin-4-ol; 3-aminopyrazolo[1,5-a]pyridin-6-ol; 3-aminopyrazolo[1,5-a]pyridin-7-ol; and their addition salts with an acid or with a base.

Mention may be made, among pyrimidine derivatives, of the compounds disclosed, for example, in Patents DE 2 359 399, JP 88-169 571, JP 05-63 124 or EP 0 770 375 or Patent Application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine or 2,5,6-triaminopyrimidine, and pyrazolopyrimidine derivatives, such as those mentioned in Patent Application FR-A-2 750 048, among which may be mentioned pyrazolo[1,5-a]pyrimidine-3,7-diamine, 2,5-dimethylpyrazolo[l,5-a]pyrimidine-3,7-diamine, pyrazolo[1,5-a]pyrimidine-3,5-diamine, 2,7-dimethylpyrazolo[1,5-a]pyrimidine-3,5-diamine, 3-aminopyrazolo[1,5-a]pyrimidin-7-ol, 3-aminopyrazolo-[1,5-a]pyrimidin-5-ol, 2-(3-aminopyrazolo[1,5-a]pyrimidin-7-ylamino)ethanol, 2-(7-aminopyrazolo[1,5-a]pyrimidin-3-ylamino)ethanol, 2-[(3-aminopyrazolo[1,5-a]pyrimidin-7-yl) (2-hydroxyethyl) amino] ethanol, 2-[(7-aminopyrazolo[1,5-a]pyrimidin-3-yl)(2-hydroxyethyl)-amino]ethanol, 5,6-dimethylpyrazolo[1,5-a]pyrimidine-3,7-diamine, 2,6-dimethylpyrazolo[1,5-a]pyrimidine-3,7-diamine, 2,5,N7,N7-tetramethylpyrazolo[1,5-a]-pyrimidine-3,7-diamine, 3-amino-5-methyl-7-(imidazolyl-propylamino)pyrazolo[1,5-a]pyrimidine, and their addition salts with an acid and their tautomeric forms, when a tautomeric equilibrium exists.

Mention may be made, among pyrazole derivatives, of the compounds disclosed in Patents DE 3 843 892 and DE 4 133 957 and Patent Applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diamino-pyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-l-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-(hydroxymethyl)pyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)-amino-1,3-dimethylpyrazole, 3,4,5-triaminopyrazole, 1-methyl-3,4,5-triaminopyrazole, 3,5-diamino-1-methyl-4-(methylamino)pyrazole, 3,5-diamino-4-(β-hydroxyethyl)-amino-1-methylpyrazole, and their addition salts with an acid.

The amount of each of the oxidation bases is generally between 0.001 and 10% by weight approximately of the total weight of the dyeing composition, preferably between 0.005 and 5%.

Mention may in particular be made, among the couplers which can be used in the composition of the present invention, of meta-phenylenediamines, meta-aminophenols, meta-diphenols, naphthalene couplers, heterocyclic couplers, and their addition salts.

Mention may be made, by way of example, of 2-methyl-5-aminophenol, 5-N-(β-hydroxyethyl)amino-2-methylphenol, 6-chloro-2-methyl-5-aminophenol, 3-aminophenol, 1,3-dihydroxybenzene, 1,3-dihydroxy-2-methylbenzene, 4-chloro-1,3-dihydroxybenzene, 2,4-diamino-l-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-(dimethylamino)benzene, sesamol, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis-(β-hydroxyethylamino)toluene, and their addition salts with an acid.

In the composition of the present invention, the amount of each of the couplers is generally between 0.001 and 10% by weight approximately of the total weight of the dyeing composition, preferably between 0.005 and 5%.

Generally, the addition salts of the oxidation bases and of the couplers which can be used in the context of the invention are chosen in particular from the addition salts with an acid, such as hydrochlorides, hydrobromides, sulphates, citrates, succinates, tartrates, lactates, tosylates, benzene-sulphonates, phosphates and acetates, and the addition salts with a base, such as sodium hydroxide, potassium hydroxide, ammonia, amines or alkanolamines.

The dyeing composition according to the invention can additionally comprise one or more direct dyes which can be chosen in particular from dyes conventionally used in direct dyeing and among which may be mentioned all the aromatic and/or nonaromatic dyes commonly used, such as nitro and amine dyes, phenols, aminophenols, naphthols, methines, azomethines, nitros, aromatic diamines, nitrosos, styryls, triarylmethanes, diarylmethanes, azos, anthraquinone and naphthoquinone dyes, porphyrins, tetraphenylporphyrins, metalloporphyrins, phthalocyanines, methinecyanines, natural dyes of carotenoid, terpenoid or flavonoid type, fluorescent molecules (fluorescein, rhodamine, coumarin, and the like), and others.

These direct dyes can be of nonionic, anionic or cationic nature.

In addition to the block copolymer, the dyeing medium, also referred to as dyeing vehicle, generally comprises water or a mixture of water and of at least one organic solvent, in order to dissolve the compounds which would not be sufficiently soluble in water. Mention may be made, as organic solvent, for example, of lower C₁-C₄ alkanols, such as ethanol and isopropanol; polyols and polyol ethers, such as 2-butoxyethanol, propylene glycol, propylene glycol monomethyl ether, diethylene glycol monoethyl ether and diethylene glycol monomethyl ether; and aromatic alcohols, such as benzyl alcohol or phenoxyethanol; and their mixtures.

The solvents are preferably present in proportions preferably of between 1 and 40% by weight approximately with respect to the total weight of the dyeing composition and more preferably still between 5 and 30% by weight approximately.

The dyeing composition in accordance with the invention can also include various adjuvants conventionally used in hair dyeing compositions, such as anionic, cationic, nonionic, amphoteric or zwitterionic surface-active agents or their mixtures, anionic, cationic, nonionic, amphoteric or zwitterionic polymers or their blends, other than the block polymers of use in the present invention, inorganic or organic thickening agents and in particular anionic, cationic, nonionic and amphoteric associative polymer thickeners, antioxidants, penetration agents, sequestering agents, fragrances, buffers, dispersing agents, conditioning agents, such as, for example, volatile or nonvolatile and modified or unmodified silicones, film-forming agents, ceramides, preservatives or opacifying agents.

The above adjuvants are generally present in an amount of, for each of them, between 0.01 and 20% by weight with respect to the weight of the composition.

Of course, a person skilled in the art will take care to choose this or these optional additional compounds so that the advantageous properties intrinsically attached to the oxidation dyeing composition in accordance with the invention are not, or not substantially, detrimentally affected by the envisaged addition or additions.

The pH of the dyeing composition in accordance with the invention is generally between 3 and 12 approximately and preferably between 5 and 11 approximately. It can be adjusted to the desired value by means of acidifying or basifying agents commonly used in dyeing keratinous fibres or alternatively using conventional buffer systems.

Mention may be made, among acidifying agents, by way of example, of inorganic or organic acids, such as hydrochloric acid, orthophosphoric acid, sulphuric acid, carboxylic acids, such as acetic acid, tartaric acid, citric acid or lactic acid, or sulphonic acids.

Mention may be made, among basifying agents, by way of example, of aqueous ammonia, alkaline carbonates, alkanolamines, such as mono-, di- and triethanolamines, and their derivatives, sodium hydroxide, potassium hydroxide and the compounds of following formula (II): 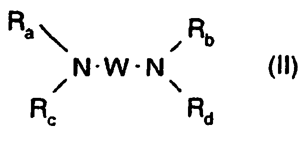 in which W is a propylene residue optionally substituted by a hydroxyl group or a C₁-C₄ alkyl radical and Rₐ, R_{b}, R_{c} and R_{d}, which are identical or different, represent a hydrogen atom or a C₁-C₄ alkyl or C₁-C₄ hydroxyalkyl radical.

The dyeing composition according to the invention can be provided in various forms, such as in the form of liquids, creams or gels or in any other form appropriate for carrying out dyeing of keratinous fibres and in particular of human hair.

The process of the present invention is a process in which the composition according to the present invention as defined above is applied to the fibres in the presence of an oxidizing agent for a time sufficient to develop the desired colouring. The colour can be developed at acidic, neutral or alkaline pH and the oxidizing agent can be added to the composition of the invention only at the time of use or it can be employed from an oxidizing composition comprising it, applied simultaneously with or sequentially to the composition of the invention.

According to a specific embodiment, the composition according to the present invention is mixed, preferably at the time of use, with a composition comprising, in a medium appropriate for dyeing, at least one oxidizing agent, this oxidizing agent being present in an amount sufficient to develop a colouring. The mixture obtained is subsequently applied to the keratinous fibres. After a leave-in time of 3 to 50 minutes approximately, preferably 5 to 30 minutes approximately, the keratinous fibres are rinsed, washed with a shampoo, rinsed again and then dried.

The oxidizing agents conventionally used for the oxidation dyeing of keratinous fibres are, for example, hydrogen peroxide, urea hydrogen peroxide, alkali metal bromates, persalts, such as perborates and persulphates, peracids and oxidase enzymes, among which may be mentioned peroxidases, 2-electron oxidoreductases, such as uricases, and 4-electron oxygenases, such as laccases. Hydrogen peroxide is particularly preferred.

The oxidizing composition can also include various adjuvants conventionally used in hair dyeing compositions and as defined above.

The pH of the oxidizing composition including the oxidizing agent is such that, after mixing with the dyeing composition, the pH of the resulting composition applied to the keratinous fibres preferably varies between 3 and 12 approximately, and more preferably still between 5 and 11. It can be adjusted to the desired value by means of acidifying or basifying agents commonly used in dyeing keratinous fibres and as defined above.

According to another embodiment, the process of the invention comprises the application to the keratinous fibres of a composition comprisng the dye precursor, in the presence of an oxidizing agent or of an oxidizing composition, for a time sufficient to develop the desired colouring, this application being followed by the application of the block copolymer.

According to one alternative form, the block copolymer is present in the oxidizing composition.

The ready-to-use composition which is finally applied to the keratinous fibres can be provided in various forms, such as in the form of liquids, of creams or of gels or in any other form appropriate for carrying out dyeing of keratinous fibres and in particular of human hair.

Another subject-matter of the invention is a dyeing multi-compartment device or "kit" in which a first compartment includes the dyeing composition of the present invention defined above and a second compartment includes an oxidizing agent.

According to a specific embodiment, the kit of the invention comprises a first compartment which comprises a dyeing composition comprising the dye precursor, a second compartment comprising the oxidizing agent and a third compartment which comprises the block copolymer. This device can be equipped with a means allowing the desired mixture to be delivered to the hair, such as the devices disclosed in Patent FR-2 586 913 on behalf of the Applicant Company.

The examples which follow serve to illustrate the invention without, however, exhibiting a limiting nature.

### EXAMPLES

### DYEING EXAMPLES

The following colouring compositions were prepared:

| | **Ex. 1** | **Ex. 2** | **Ex. 3** | **Ex. 4** |
|---|---|---|---|---|
| Hexylene glycol | 12.5 g | | | |
| Propylene glycol | 12.5 g | | | |
| Ethanol | 10 g | | | |
| Sodium metabisulphite | 0.30 g | | | |
| para-Phenylenediamine | 0.49 g | | | |
| 1-Methyl-2-hydroxy-4-aminobenzene | 0.55 g | | | |
| Polymer 1 (comp.) | 1 g | | | |
| Polymer 2 | | 1 g | | |
| Polymer 3 | | | 1 g | |
| Polymer 4 | | | | 1 g |
| Polymer 6 | | | | |
| Aqueous ammonia | 10 g | 10 g | 10 g | 10 g |
| Water | q.s. for 100 g | q.s. for 100 g | q.s. for 100 g | q.s. for 100 g |

At the time of use, these compositions are mixed weight for weight with an oxidizing milk assaying 20 volumes of hydrogen peroxide, and then the various mixtures obtained are applied to locks of highly sensitized hair. The leave-in time is set at 30 minutes at ambient temperature. At the end of the leave-in time, the locks are shampooed, rinsed and dried.

Each lock is evaluated before and after dyeing in the L*a*b* system using a CM 2002 Minolta^{®} spectrophotometer (D65 illuminant).

In the L*a*b* system, the three parameters respectively denote the intensity (L*), the hue (a*) and the saturation (b*). According to this system, the higher the value of L, the lighter or less intense the colour. Conversely, the lower the value of L, the darker or more intense the colour. a* and b* indicate two axes of colours: a* indicates the axis of green/red colour and b* the axis of blue/yellow colour.

The colorimetric results given by L* are as follows:

| | **L*** |
|---|---|
| **Locks before colouring** | **56.51** |
| **Example 1** | 34.1 |
| **Example 2** | 19.50 |
| **Example 3** | 20.62 |
| **Example 4** | 16.80 |

These results show the superiority of the colouring with the composition of the present invention.

| | **BLOCK POLYMER** | **CHARGE** | **CHARACTERISTICS** |
|---|---|---|---|
| Polymer 1 (comparative) | PPG-14 Laureth-60 hexyldicarbamate (Dapral T sold by Akzo) | **Nonionic** | Carbamic diester of oxypropylenated (14 mol) and oxyethylenated (60 mol) lauryl alcohol |
| Polymer 2 (invention) | **P**oly(**S**tyrene/ **A**crylic **A**cid) | **Anionic** | **PS**(1500)- **PAA**(44 000) |
| Polymer 3 (invention) | **P**oly(**S**tyrene/ **A**crylic **A**cid) | **Anionic** | **PS**(4500)- **PAA**(22 800) |
| Polymer 4 (invention) | **P**oly (**S**tyrene/β N-methyl-4- **V**inyl-**P**yridinium iodide) | **Cationic** | **PS**(18 600)- **P4VPQ**(131 300) |

## Claims

1. Dyeing composition comprising, in an appropriate medium,
• at least one dye precursor, and
• at least one water-soluble or water-dispersible amphiphilic linear diblock copolymer comprising at least one hydrophilic block and at least one hydrophobic block without a fatty chain of more than 8 carbon atoms, the hydrophilic block or blocks representing at least 30% by weight of the block copolymer the hydrophobic block being formed of water-insoluble monomers chosen from vinylaromatic monomers and the hydrophilic block is formed of anionic monomers chosen from carboxylic acids comprising ethylenic unsaturation or cationic monomers from N-methyl-4-vinyl pyridinium, with the exception of block copolymers of ethylene oxide and of propylene oxide, block copolymers comprising urethane units and block copolymers comprising siloxane units.

2. Composition according to Claim 1, in which the hydrophilic block represent at least 60% by weight of the block copolymer.

3. Composition according to Claim 1, in which the hydrophilic blocks comprise at most 25 mol% of one or more water-insoluble monomers according to Claim 1.

4. Composition according to Claim 1 or 2, in which the hydrophobic block or blocks comprise at most 25 mol% of one or more water-soluble monomers according to any one of Claim 1.

5. Composition according to any one of the preceding claims, in which the block polymer or polymers are present in the composition of the present invention in an amount of between 0.01 and 20% by weight of active material, preferably between 0.1 and 5% by weight of active material, with respect to the weight of the dyeing composition.

6. Composition according to any one of the preceding claims, comprising an oxidation base chosen from para-phenylenediamines, bisphenylalkylenediamines, bis para-aminophenol, para-aminophenols, ortho-aminophenols, heterocyclic bases and their addition salts.

7. Composition according to any one of the preceding claims, comprising a coupler chosen from meta-phenylenediamines, meta-aminophenols, metadiphenols, naphthalene couplers, heterocyclic couplers, and their addition salts.

8. Composition according to Claim 5, in which the amount of each of the oxidation bases is between 0.001 and 10% by weight approximately of the total weight of the dyeing composition.

9. Composition according to Claim 6, in which the amount of each of the couplers is between 0.001 and 10% by weight approximately of the total weight of the dyeing composition.

10. Process for the oxidation dyeing of keratinous fibres, comprising the application to the fibres of a dye composition as defined in any one of Claims 1 or 2 in the presence of an oxidizing agent for a time sufficient to develop the desired colouring.

11. Process according to Claim 9, in which the oxidizing agent is chosen from hydrogen peroxide, urea hydrogen peroxide, alkali metal bromates, persalts, peracids and oxidase enzymes.

12. Process for the oxidation dycing of keratinous fibres, comprising the application of a dyeing compositions comprising a dye precursor as defined in Claims 1 to 8, in the presence of an oxidizing agent or of an oxidizing composition, for a time sufficient to develop the desired colouring, this application being followed by the application of the block copolymer as defined in Claims 1 to 4.

13. Process according to Claim 11, in which the block copolymer is present in the,oxidizing composition.

14. Multi-compartment device, in which a first compartment comprises a dyeing composition as defined in any one of Claims 1 to 8 and a second compartment comprises an oxidizing agent.

15. Multi-compartment device, in which a first compartment comprises a dyeing composition comprising at least one dye precursor, a second compartment comprising the oxidizing agent and a third compartment which comprises the block copolymer, the dye precursor and the block copolymer being as defined according to any one of Claims 1 to 8.

16. Use of the composition defined in Claims 1 to 8 for dyeing keratinous fibres.

## Patentansprüche

1. Farbmittelzusammensetzung, die in einem geeigneten Medium enthält:
■ mindestens ein Farbstoffvorprodukt, und
■ mindestens ein wasserlösliches oder in Wasser dispergierbares, amphiphiles lineares Zweiblockcopolymer, das mindestens einen hydrophilen Block und mindestens einen hydrophoben Block ohne Fettkette mit mehr als 8 Kohlenstoffatomen aufweist, wobei der hydrophile Block oder die hydrophilen Blöcke mindestens 30 Gew.-% des Blockcopolymers ausmachen, wobei der hydrophobe Block aus in Wasser unlöslichen Monomeren gebildet ist, die unter den vinylaromatischen Monomeren ausgewählt sind, und der hydrophile Block aus anionischen Monomeren, die unter den Carbonsäuren ausgewählt sind, die eine ethylenisch ungesättigte Bindung enthalten, oder kationischen N-Methyl-4-vinyl-pyridiniummonomeren gebildet ist, wobei Blockcopolymere von Ethylenoxid und Propylenoxid, Blockcopolymere, die Urethaneinheiten enthalten, und Blockcopolymere, die Siloxaneinheiten enthalten, ausgenommen sind.

2. Zusammensetzung nach Anspruch 1, wobei der hydrophile Block mindestens 60 Gew.-% des Blockcopolymers ausmacht.

3. Zusammensetzung nach Anspruch 1 oder 2, wobei die hydrophilen Blöcke höchstens 25 Mol-% eines oder mehrerer wasserlöslicher Monomere gemäß Anspruch 1 umfassen.

4. Zusammensetzung nach Anspruch 1 oder 3, wobei der oder die hydrophoben Blöcke höchstens 25 Mol-% eines oder mehrerer wasserlöslicher Monomere gemäß Anspruch 1 umfassen.

5. Zusammensetzung nach einem der vorhergehenden Ansprüche, wobei das Blockpolymer oder die Blockpolymere in der erfindungsgemäßen Zusammensetzung in einem Mengenanteil der wirksamen Substanz im Bereich von 0,01 bis 20 Gew.-% und vorzugsweise im Bereich von 0,1 bis 5 Gew.-%, bezogen auf das Gewicht der Farbmittelzusammensetzung, enthalten sind.

6. Zusammensetzung nach einem der vorhergehenden Ansprüche, die eine Oxidationsbase enthält, die unter den p-Phenylendiaminen, Bisphenylalkylendiaminen, Bis-*p*-Aminophenol, *p-*Aminophenolen, *o*-Aminophenolen und den heterocyclischen Basen und deren Additionssalzen ausgewählt ist.

7. Zusammensetzung nach einem der vorhergehenden Ansprüche, die einen Kuppler enthält, der unter den m-Phenylendiaminen, m-Aminophenolen, *m*-Dihydroxybenzolen, Naphthalinkupplern und den heterocyclischen Kupplern und deren Additionssalzen ausgewählt ist.

8. Zusammensetzung nach Anspruch 6, wobei der Mengenanteil jeder Oxidationsbase im Bereich von etwa 0,001 bis 10 Gew.-% des Gesamtgewichts der Farbmittelzusammensetzung liegt.

9. Zusammensetzung nach Anspruch 7, wobei der Mengenanteil jedes Kupplers im Bereich von etwa 0,001 bis 10 Gew.-% des Gesamtgewichts der Farbmittelzusammensetzung liegt.

10. Verfahren zum oxidativen Färben von Keratinfasern, das umfasst, eine Farbmittelzusammensetzung, wie sie in einem der Ansprüche 1 bis 9 definiert ist, in Gegenwart eines Oxidationsmittels während einer Zeitspanne, die zur Bildung der gewünschten Färbung ausreichend ist, auf die Fasern aufzubringen.

11. Verfahren nach Anspruch 10, wobei das Oxidationsmittel unter Wasserstoffperoxid, Harnstoffperoxid, Alkalimetallbromaten, Salzen von Persäuren, Persäuren und Oxidasen ausgewählt ist.

12. Verfahren zum oxidativen Färben von Keratinfasern, das umfasst, eine Farbmittelzusammensetzung, die ein wie in den Ansprüchen 1 bis 9 definiertes Farbstoffvorprodukt enthält, in Gegenwart eines Oxidationsmittels oder einer oxidierenden Zusammensetzung während einer Zeitspanne, die zur Bildung der gewünschten Färbung ausreichend ist, auf die Fasern aufzubringen, worauf ein wie in den Ansprüchen 1 bis 5 definiertes Blockcopolymer aufgetragen wird.

13. Verfahren nach Anspruch 12, wobei das Blockcopolymer in der oxidierenden Zusammensetzung enthalten ist.

14. Vorrichtung mit mehreren Abteilungen, wobei eine erste Abteilung eine Farbmittelzusammensetzung nach einem der Ansprüche 1 bis 9 enthält und eine zweite Abteilung ein Oxidationsmittel enthält.

15. Vorrichtung mit mehreren Abteilungen, wobei eine erste Abteilung eine Farbmittelzusammensetzung enthält, die mindestens ein Farbstoffvorprodukt umfasst, eine zweite Abteilung ein Oxidationsmittel enthält und eine dritte Abteilung ein Blockpolymer enthält, wobei das Farbstoffvorprodukt und das Blockcopolymer wie in einem der Ansprüche 1 bis 9 definiert vorliegen.

16. Verwendung der in den Ansprüchen 1 bis 9 definierten Zusammensetzung zum Färben von Keratinfasern.

## Revendications

1. Composition de coloration comprenant, dans un milieu approprié,
• au moins un précurseur de colorant, et
• au moins un copolymère diblocs linéaire amphiphile hydrosoluble ou hydrodispersible comprenant au moins un bloc hydrophile et au moins un bloc hydrophobe sans chaîne grasse de plus de 8 atomes de carbone, le ou les blocs hydrophiles représentant au moins 30 % en poids du copolymère bloc, le bloc hydrophobe étant formé de monomères insolubles dans l'eau, choisis parmi les monomères vinylaromatiques et le bloc hydrophile étant formé de monomères anioniques choisis parmi les acides carboxyliques comprenant une insaturation éthylénique ou les monomères cationiques de N-méthyl-4-vinylpyridinium, à l'exception des copolymères blocs d'oxyde d'éthylène et d'oxyde de propylène, des copolymères blocs comprenant des motifs uréthanes et des copolymères blocs comprenant des motifs siloxanes.

2. Composition selon la revendication 1, **caractérisée en ce que** le bloc hydrophile représente au moins 60 % en poids du copolymère bloc.

3. Composition selon la revendication 1 ou 2, **caractérisée en ce que** les blocs hydrophiles comprennent au plus 25 % en moles d'un ou plusieurs monomères insolubles dans l'eau selon la revendication 1.

4. Composition selon la revendication 1 ou 3, **caractérisée en ce que** le ou les blocs hydrophobes comprennent au plus 25 % en moles d'un ou plusieurs monomères hydrosolubles selon la revendication 1.

5. Composition selon l'une quelconque des revendications précédentes, **caractérisée en ce que** le ou les polymères blocs sont présents dans la composition de la présente invention dans une quantité comprise entre 0,01 et 20 % en poids de la matière active, de préférence entre 0,1 et 5 % en poids de la matière active, par rapport au poids de la composition de coloration.

6. Composition selon l'une quelconque des revendications précédentes, comprenant une base d'oxydation choisie parmi les paraphénylènediamines, les bisphénylalkylènediamines, le bis-para-aminophénol, les para-aminophénols, les ortho-aminophénols, les bases hétérocycliques et leurs sels d'addition.

7. Composition selon l'une quelconque des revendications précédentes, comprenant un coupleur choisi parmi les méta-phénylènediamines, les méta-aminophénols, les méta-diphénols, les coupleurs naphtaléniques, les coupleurs hétérocycliques et leurs sels d'addition.

8. Composition selon la revendication 6, **caractérisée en ce que** la quantité de chacune des bases d'oxydation est comprise entre 0,001 et 10 % en poids approximativement du poids total de la composition de coloration.

9. Composition selon la revendication 7, **caractérisée en ce que** la quantité de chacun des coupleurs est comprise entre 0,001 et 10 % en poids approximativement du poids total de la composition de coloration.

10. Procédé de coloration d'oxydation des fibres kératiniques, comprenant l'application aux fibres d'une composition de coloration telle que définie dans l'une quelconque des revendications 1 à 9, en présence d'un agent d'oxydation pendant un temps suffisant pour développer la coloration désirée.

11. Procédé selon la revendication 10, **caractérisé en ce que** l'agent d'oxydation est choisi parmi le peroxyde d'hydrogène, l'urée-peroxyde d'hydrogène, les bromates de métaux alcalins, les persels, les peracides et les enzymes oxydases.

12. Procédé de coloration d'oxydation des fibres kératiniques, comprenant l'application d'une composition de coloration comprenant un précurseur de colorant telle que définie dans les revendications 1 à 9, en présence d'un agent d'oxydation ou d'une composition oxydante, pendant un temps suffisant pour développer la coloration désirée, cette application étant suivie de l'application du copolymère bloc tel que défini dans les revendications 1 à 5.

13. Procédé selon la revendication 12, **caractérisé en ce que** le copolymère bloc est présent dans la composition oxydante.

14. Dispositif à plusieurs compartiments, **caractérisé en ce qu'**un premier compartiment comprend une composition de coloration telle que définie dans l'une quelconque des revendications 1 à 9 et un deuxième compartiment comprend un agent d'oxydation.

15. Dispositif à plusieurs compartiments, **caractérisé en ce qu'**un premier compartiment comprend une composition de coloration comprenant au moins un précurseur de colorant, un deuxième compartiment comprenant l'agent d'oxydation et un troisième compartiment qui comprend le copolymère bloc, le précurseur de colorant et le copolymère bloc étant tels que définis dans l'une quelconque des revendications 1 à 9.

16. Utilisation de la composition définie dans les revendications 1 à 9, pour la coloration des fibres kératiniques.
